# EUROPÄISCHE PATENTSCHRIFT

(11) **EP 1 450 709 B1**
(45) Veröffentlichungstag und Bekanntmachung des Hinweises auf die Patenterteilung: **22.03.2006**
(21) Anmeldenummer: 01274861.2
(22) Anmeldetag: 04.12.2001
(51) Int. Cl.: A61B 17/86, A61B 17/88

(54) **KNOCHENSCHRAUBE**
BONE SCREW
VIS A OS

(43) Veröffentlichungstag der Anmeldung: 01.09.2004
(73) Patentinhaber: Synthes AG Chur, 7002 Chur (CH)
(72) Erfinder: FRENK, André, CH-4805 Brittnau (CH); BEUTTER, Florian, CH-4500 Solothurn (CH); CICOIRA, Franco, CH-2545 Selzach (CH)
(74) Vertreter: Lusuardi, Werther
(86) Internationale Anmeldenummer: PCT/CH2001/000698
(87) Internationale Veröffentlichungsnummer: WO 2003/047444

(56) Entgegenhaltungen:
- EP-A- 0 491 211
- EP-A- 0 856 293
- US-A- 4 463 753
- US-A- 5 375 956

## Beschreibung

Die Erfindung bezieht sich auf eine Knochenschraube zur Verbindung zweier Knochenfragmente, gemäss dem Oberbegriff des Patentanspruchs 1, und auf eine Vorrichtung zur Reposition, Kompression und/oder Fixation von Knochenfragmenten, gemäss dem Patentanspruch 2.

Knochenschrauben werden in der Osteosynthese vielfältig angewendet, beispielsweise zur Reponierung von Knochenfragmenten, als Kompressionsschrauben oder zur Fixation von Knochenfragmenten.

Eine Knochenschraube mit zwei axial endständig angeordneten Gewindesegmenten und einem mittleren gewindelosen Segment ist aus der US 5,019,079 ROSS bekannt. Der Durchmesser des mittleren Segmentes entspricht im wesentlichen dem Aussendurchmesser des Aussengewindes am distalen Gewindesegment, ist aber grösser als der Kerndurchmesser des Aussengewindes am proximalen Gewindesegment, so dass das mittlere Segment zur lateralen Stabilisierung der beiden Knochenfragmente bei der Fraktur benützt werden kann. Nachteilig an dieser Ausführung von Knochenschrauben ist, dass die beiden Aussengewinde verschiedene Gewindesteigungen aufweisen, so dass die verschiedenen Schritte bei der Implantation, die Reposition der Knochenfragmente, die Kompression der Knochenfragmente und das Versenken des Schraubenkopfes nicht getrennt voneinander durchgeführt werden können.

Hier will die Erfindung Abhilfe schaffen. Der Erfindung liegt die Aufgabe zugrunde, eine Knochenschraube zu schaffen, welche während der Implantation eine separate Ausführung der Reposition von Knochenfragmenten, der Kompression von Knochenfragmenten und des Versenkens des Schraubenkopfes ermöglicht.

Die Erfindung löst die gestellte Aufgabe mit einer Knochenschraube, welche die Merkmale des Anspruchs 1 aufweist, und mit einer Vorrichtung zur Reposition Kompression und Fixation, welche die Merkmale des Anspruchs 2 aufweist.

Die erfindungsgemässe Knochenschraube umfasst im wesentlichen zwei koaxial zur Längsachse und endständig an der Knochenschraube angeordnete Gewindesegmente, wobei die Gewindesteigungen S_{V} und S_{H} des vorderen respektive des hinteren Gewindesegmente gleich sind. Damit ist erreichbar, dass nach erfolgter Reposition und Kompression der beiden Knochenfragmente, wozu nur das vordere Gewindesegment im distalen Knochenfragment eingeschraubt ist während das hintere Gewindesegment beispielsweise in einem Implantationsinstrument eingeschraubt ist und noch nicht in das proximale Knochenfragment eingeschraubt ist, die Knochenschraube weiter in die Knochenfragmente eingeschraubt werden kann, bis das hintere Gewindesegment ebenfalls vollständig im proximalen Knochenfragment versenkt ist, ohne dass dabei die Position der Knochenfragmente relativ zueinander verändert wird und ohne dass die Kompression der beiden Knochenfragmente verändert wird. Die beiden Gewindesegmente sind so ausgebildet, dass der Aussendurchmesser des vorderen Gewindesegmentes kleiner als der Kemdurchmesser des Aussengewindes am hinteren Gewindesegment ist. Eine Knochenschraube mit diesen Merkmalen ist aus der EP 0 491 211 bekannt, worauf der Oberbegriff des Anspruchs 1 basiert.

Die durch die Erfindung erreichten Vorteile sind im wesentlichen darin zu sehen, dass dank der erfindungsgemässen Knochenschraube und der erfindungsgemässen Vorrichtung wegen der gleichen Gewindesteigung der Aussengewinde am vorderen Gewindesegment und am hinteren Gewindesegment die Operationsschritte
- Reposition der Knochenfragmente;
- Kompression der Knochenfragmente; und
- Versenken des Schraubenkopfes
separat und kontrolliert ausgeführt werden können.

Durch die Ausgestaltung des hinteren Gewindesegmentes mit einem grösseren Kerndurchmesser als der Aussendurchmesser des vorderen Gewindesegmentes ist ein Interagieren des vordaren Gewindesegmentes mit dem bereits für das hintere Gewindesegment in den Knochenfragmenten geschnittenen Gewinde vermeidbar. Vorzugsweise sind die Aussengewinde am vorderen und am hinteren Gewindesegment als selbstschneidende Gewinde ausgeführt.

Der erfindungsgemässen Knochenschraube umfasst zwischen den beiden Gewindesegmenten ein mittleres, gewindeloses Segment, welches einen Aussendurchmesser aufweist, der kleiner oder gleich dem Kerndurchmesser des Aussengewindes am vorderen Gewindesegment ist. Damit ist der Vorteil erreichbar, dass das vordere Gewindesegment vollständig in das distale Knochenfragment einschraubbar ist und für die Ausführung einer Reposition und Kompression der Knochenfragmente die Bohrung im proximalen Knochenfragment gegenüber der Bohrung im distalen Knochenfragment nicht vergrössert werden muss. Gegenüber einer Ausführungsform der Knochenschraube, deren vorderes Gewindesegment axial direkt an das hintere Gewindesegment anschliesst und bei der die Bohrung im proximalen Knochenfragment vergrössert werden müsste, damit das vordere Gewindesegment nur in das distale Knochenfragment einschraubbar ist, ist zudem eine höhere Stabilität der Verbindung zwischen der Knochenschraube und dem proximalen Knochenfragment erreichbar.

Die erfindungsgemässe Vorrichtung dient zur Reposition, Kompression und Fixation von Knochenfragmenten mit einer Knochenschraube und umfasst im wesentlichen die Schraube und ein chirurgisches Implantationsinstrument, welches vorzugsweise eine das Implantationsinstrument koaxial durchdringende Zentralbohrung zur Durchführung eines Schraubendrehers aufweist. Ferner ist die Zentralbohrung vom vorderen Ende des Implantationsinstrumentes bis zu einer Tiefe T erweitert, so dass in der Tiefe T eine Schulter gebildet wird. Im erweiterten Teil der Zentralbohrung ist ein Innengewinde angebracht, welches zum Aussengewinde des hinteren Gewindesegmentes der Knochenschraube komplementär ist, so dass das hintere Gewindesegment der Knochenschraube bis zur Tiefe T in die Zentralbohrung einschraubbar ist. Die Tiefe T ist so gewählt, dass T ≥ L ist, wobei L die Länge des hinteren Gewindesegmentes der Knochenschraube ist. Damit ist erreichbar, dass das hintere Gewindesegment der Knochenschraube vollständig in die Zentralbohrung des Implantationsinstrumentes

Die Erfindung und Weiterbildungen der Erfindung werden im folgenden anhand der teilweise schematischen Darstellungen mehrerer Ausführungsbeispiele noch näher erläutert.

Es zeigen:
Fig. 1 eine Ansicht einer Ausführungsform der efindungsgemässen Knochenschraube; und
Fig. 2 einen Längsschnitt durch die erfindungsgemässe Vorrichtung mit einer Knochenschraube gemäss der in Fig. 1 dargestellten Ausführungsform, einem Implantationsinstrument und einem Schraubendreher.

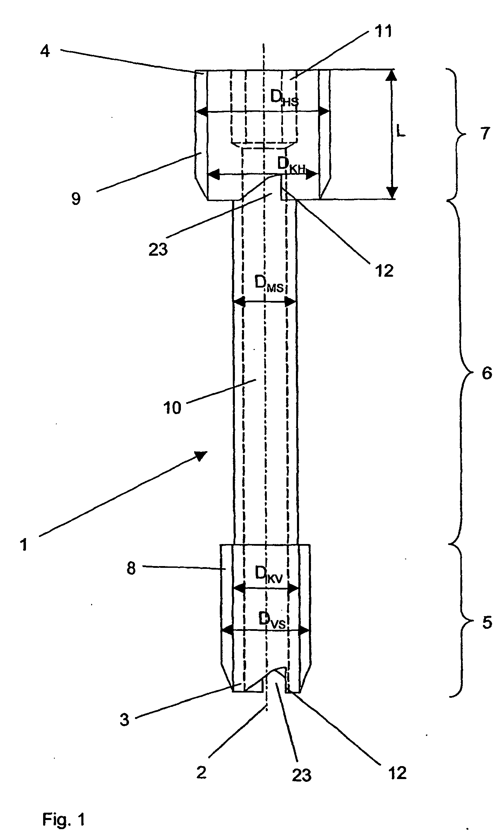

In Fig. 1 ist die bevorzugte Ausführungsform der erfindungsgemässen Knochenschraube 1 dargestellt. Diese umfasst ein hinteres Gewindesegment 7 mit einem Aussengewinde 9, welches einen Kemdurchmesser D_{KH}, einen Aussendurchmesser D_{HS} und eine Gewindesteigung S_{H} aufweist, ein mittleres, gewindeloses Segment 10 mit einem Aussendurchmesser D_{MS}, welches koaxial zur Längsachse 2 an das hintere Gewindesegment 7 angrenzt, und ein vorderes Gewindesegment 5 mit einem Aussengewinde 8, welches einen Kemdurchmesser D_{KV}, einen Aussendurchmesser D_{VS} und eine Gewindesteigung S_{V} aufweist. Die beiden Gewindesegmente 5;7 haben unterschiedliche Durchmesser, d.h. der Kemdurchmesser D_{KH} des hinteren Gewindesegmentes 7 ist grösser oder gleich dem Aussendurchmesser D_{VS} des vorderen Gewindesegmentes 5. Die Gewindesteigungen der beiden Aussengewinde 8;9 sind jedoch gleich. Der Aussendurchmesser D_{MS} des mittleren Segmentes 6 ist kleiner oder gleich dem Kemdurchmesser D_{KV} des vorderen Gewindesegmentes 5. Ferner sind am vorderen Ende 3 der Knochenschraube 1 und am Übergang zwischen dem hinteren Gewindesegment 7 und dem mittleren Segment 6 mehrere auf dem Umfang der beiden Gewindesegmente 5;7 verteilte und axial ausgerichtete Einkerbungen 23 mit zur Längsachse 2 im wesentlichen parallelen Schneidkanten 12 angeordnet, so dass die beiden Aussengewinde 8;9 selbstschneidend ausgebildet sind. Am hinteren Ende 4 der Knochenschraube 1 sind koaxial die Mittel 11 zur Aufnahme eines Schraubendrehers, beispielsweise als Innensechskant, Torx oder Phillips angeordnet. Zudem ist die Knochenschraube 1 mit einer vom vorderen Ende 3 bis zum hinteren Ende 4 durchgehenden Zentralbohrung 10 ausgestattet, welche beispielsweise zur Aufnahme eines Fuhrüngsdrahtes (nicht gezeichnet) dient.

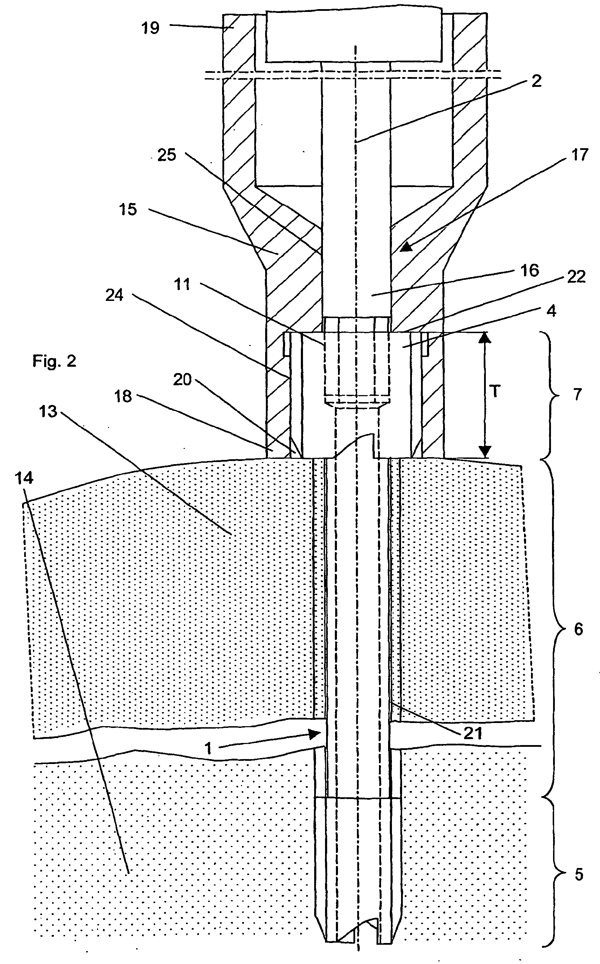

In Fig. 2 dargestellt ist die erfindungsgemässe Vorrichtung mit einer Knochenschraube 1, deren hinteres Gewindesegment 7 vollständig im Implantationsinstrument 15 eingeschraubt ist und deren vorderes Gewindesegment 5 vollständig in das distale Knochenfragment 14 eingeschraubt ist. Das Implantationsinstrument 15 umfasst eine durchgehende Zentralbohrung 17, welche vom vorderen Ende 18 bis zu einer Tiefe T erweitert ist und in diesem erweiterten Teil 24 ein zum Aussengewinde 9 komplementäres Innengewinde 20 aufweist. In der Tiefe T ist zwischen dem erweiterten Teil 24 der Zentralbohrung 17 und dem engeren Teil 25 der Zentralbohrung 17 eine Schulter 22 angebracht, woran das hintere Ende 4 der Knochenschraube 1 bei vollständig in das Implantationsinstrument 15 eingeschraubtem hinteren Gewindesegment 7 ansteht. Durch den engeren Teil 25 der Zentralbohrung 17 ist vom hinteren Ende 19 des Implantationsinstrumentes 15 her ein Schraubendreher 17 durchführbar, so dass der Schraubendreher 17 in die am hinteren Ende 4 der Knochenschraube 1 angeordneten Mittel 11 zur Aufnahme eines Schraubendrehers eingeführt werden kann und die Knochenschraube 1 mittels des Schraubendrehers 16 relativ zum Implantationsinstrument 15 rotierbar ist.

Zur Reposition, Kompression und Fixation der beiden Knochenfragmente 13;14 wird zuerst eine das proximale Knochenfragment 13 durchdringende und in das distale Knochenfragment 14 eindringende Bohrung 21 angebracht, deren Durchmesser dem Kerndurchmesser D_{KV} (Fig. 1) des Aussengewindes 8 am vorderen Gewindesegment 5 der Knochenschraube 1 entspricht.

Bei Beginn des Implantationsvorganges wird das hintere Gewindesegment 7 der Knochenschraube 1 vollständig und bis zur Tiefe T in das Innengewinde 20 in der Zentralbohrung 17 des Implantationsinstrumentes 15 eingeschraubt. Die Knochenschraube 1 wird dann durch Drehen des Implantationsinstrumentes 15 um die Längsachse 2 in die vorgebohrten Bohrungen 21 in den beiden Knochenfragmenten 13;14 eingedreht. Da das hintere Gewindesegment 7 der Knochenschraube 1 vollständig im Implantationsinstrument 15 aufgenommen wird, kann das Aussengewinde 9 des hinteren Gewindesegmentes 7 nicht in das proximale Knochenfragment 13 eingreifen, so dass beim Drehen des Implantationsinstrumentes 15 lediglich das vordere Gewindesegment 5 der Knochenschraube 1 in das distale Knochenfragment 14 eingeschraubt wird. Das vordere Ende 18 des Implantationsinstrumentes 15 übernimmt in dieser Phase die Aufgabe eines Schraubenkopfes, so dass nachdem die Knochenschraube 1 so weit in die beiden Knochenfragmente 13;14 eingebracht ist, bis das vordere Ende 18 des Implantationsinstrumentes 15 am proximalen Knochenfragment 14 anliegt, durch weiteres Drehen des Implantationsinstrumentes 15 die beiden Knochenfragmente 13;14 aufeinander zu bewegt werden. Sobald die beiden Knochenfragmente 13; 14 einander berühren beginnt der Kompressionsvorgang der beiden Knochenfragmente 13;14. Sobald durch weiteres Drehen des Implantationsinstrumentes 15 die gewünschte Kompression der beiden Knochenfragmente 13;14 erreicht ist, wird der Schraubendreher 16 durch die Zentralbohrung 17 im Implantationsinstrument 15 in die Mittel 11 zur Aufnahme des Schraubendrehers eingefügt und mit dem Schraubendreher 16 die Knochenschraube 1 weiter gedreht, so dass bei festgehaltenem Implantationsinstrument 15 die Knochenschraube 1 aus dem Innengewinde 20 am vorderen Ende 18 des Implantationsinstrumentes 15 herausgedreht und das hintere Gewindesegment 7 in das proximale Knochenfragment 13 eingeschraubt wird bis dieses vollständig unter die Oberfläche des proximalen Knochenfragmentes 13 gebracht ist. Da während dieses letzten Vorganges die beiden Knochenfragmente 13;14 relativ zueinander nicht bewegt werden, ist nach dem Versenken des hinteren Gewindesegmentes 7 im proximalen Knochenfragment 13 die Kompression unverändert.

Die Knochenschraube 1 wird bevorzugt dort eingesetzt, wo ein Schraubenkopf stören würde, z.B. Gelenksnahe Frakturen, intraartikuläre Fixationen wie Scaphoid-Frakturen, Kleinfragmente, Sehnen-, Nerven- sowie gefässnahe Fixationen.

## Patentansprüche

1. Knochenschraube (1) zur Verbindung zweier Knochenfragmente mit
A) einer Längsachse (2), einem vorderen Ende (3), einem hinteren Ende (4) und Mitteln (11) zur Aufnahme eines Schraubendrehers (16) am hinteren Ende (4); sowie mit
B) einem zur Längsachse (2) koaxial und axial endständig angeordneten, vorderen Gewindesegment (5), welches ein Aussengewinde (8) mit einem Kemdurchmesser D_{KV}, einem Aussendurchmesser Dvs und einer Gewindesteigung Sv umfasst;
C) einem zur Längsachse (2) koaxial und axial endständig angeordneten, hinteren Gewindesegment (7), welches ein Aussengewinde (9) mit einem Kemdurchmesser D_{KH}, einem Aussendurchmesser D_{HS} und einer Gewindesteigung S_{H} umfasst, wobei
D) der Aussendurchmesser Dvs des vorderen Gewindesegmentes (5) kleiner oder gleich dem Kerndurchmesser D_{KH} des hinteren Gewindesegmentes (7) ist; und
E) die Gewindesteigung Sv des vorderen Gewindesegmentes (5) und die Gewindesteigung S_{H} des hinteren Gewindesegmentes (4) gleich sind,
**dadurch gekennzeichnet, dass**
F) die Knochenschraube (1) zwischen den beiden Gewindesegmenten (5;7) ein ebenfalls zur Längsachse (2) koaxiales, mittleres Segment (6) mit einem konstanten Aussendurchmesser D_{MS} umfasst, wobei
G) der Aussendurchmesser D_{MS} kleiner oder gleich dem Kemdurchmesser des Aussengewindes (8) am vorderen Gewindesegment (5) ist; und wobei
H) das mittlere Segment (6) gewindelos ausgebildet ist.

2. Vorrichtung zur Reposition, Kompression und Fixation von Knochenfragmenten unfassend mindestens eine Knochenschraube (1) nach Anspruch 1,
**dadurch gekennzeichnet, dass**
A) die Vorrichtung ein zylindrisches oder prismatisches chirurgisches Implantationsinstrument (15) umfasst, welches eine koaxiale, durchgehende Zentralbohrung (17), ein vorderes Ende (18) und ein hinteres Ende (19) aufweist; und
B) die Zentralbohrung (17) einen sich vom vorderen Ende (18) des Instrumentes (15) bis zu einer Tiefe T erstreckenden, erweiterten Teil (24) mit einem Innengewinde (20) aufweist, welches zum Aussengewinde (9) am hinteren Segment (7) der Knochenschraube (1) komplementär ist und die Tiefe T ≥ L ist, wobei L die Länge des hinteren Gewindesegmentes (7) der Knochenschraube (1) ist.

## Claims

1. Bone screw (1) for connecting two bone fragments, including
A) a longitudinal axis (2), a front end portion (3), a rear end portion (4), and means (11) for receiving a screwdriver (16) on the rear end portion (4); further including
B) a threaded front segment (5) extending coaxially to the longitudinal axis (2) and located axially in the front end portion, comprising an external screw thread (8) having a core diameter D_{KV}, an outside diameter D_{VS}, and a thread pitch S_{V};
C) a threaded rear segment (7) extending coaxially to the longitudinal axis (2) and located axially in the rear end portion, comprising an external screw thread (9) having a core diameter D_{KH}, an outside diameter D_{HS}, and a thread pitch S_{H};
**characterised in that**
D) the outside diameter D_{VS} of the threaded front segment (5) is smaller than, or equal to the core diameter D_{KH} of the threaded rear segment (7); and
E) the thread pitch S_{V} of the threaded front segment (5) and the thread pitch S of the threaded rear segment (4) are identical,
**characterised in that**
F) the bone screw (1) comprises an intermediate segment (6) arranged between the two threaded segments (5;7) and equally extending coaxially to the longitudinal axis (2), having a constant outside diameter D_{MS}; whereby
G) said outside diameter D_{MS} is smaller than, or equal to the core diameter of the external screw thread (8) formed in the threaded front segment (5); and whereby
H) said intermediate segment (6) has no thread.

2. A device for the reduction, compression, and fixation of bone fragments comprising at least one bone screw (1) according to claim 1
**characterized in that**
A) said device comprises a cylindrical or prismatic, surgical implantation instrument (15) which comprises a coaxial, continuous central bore (17), a front end portion (18), and a rear end portion (19); and
B) the central bore (17) has an enlarged portion (24) extending from the front end portion (18) of the instrument (15) to a depth T and provided with an internal screw thread (20) which is complementary to the external screw thread (9) formed in the rear segment (7) of the bone screw (1), whereby the depth T is ≥ L, L being the length of the threaded rear segment (7) of the bone screw (1).

## Revendications

1. Vis d'ostéosynthèse (1) permettant d'assembler deux fragments osseux, laquelle comprend
A) un axe longitudinal (2), une extrémité avant (3), une extrémité arrière (4) et des moyens (11) destinés à recevoir un tournevis (16) au niveau de l'extrémité arrière (4); et laquelle comprend
B) un segment fileté avant (5) disposé coaxialement à l'axe longitudinal (2) et axialement de manière terminale, lequel comprend un filetage extérieur (8) présentant un diamètre de noyau D_{KV}, un diamètre extérieur D_{VS} et un pas de filetage Sv;
C) un segment fileté arrière (7) disposé coaxialement à l'axe longitudinal (2) et axialement de manière terminale, lequel comprend un filetage extérieur (9) présentant un diamètre de noyau D_{KH}, un diamètre extérieur D_{HS} et un pas de filetage S_{H},
D) le diamètre extérieur Dvs du segment fileté avant (5) étant inférieur ou égal au diamètre de noyau D_{KH} du segment fileté arrière (7); et
E) le pas de filetage S_{V} du segment fileté avant (5) et le pas de filetage S_{H} du segment fileté arrière (4) étant égaux,
**caractérisée en ce que**
F) la vis d'ostéosynthèse (1) comprend, entre les deux segments filetés (5;7), un segment central (6) disposé également de manière coaxiale à l'axe longitudinal (2) et présentant un diamètre extérieur constant D_{MS},
G) le diamètre extérieur D_{MS} étant inférieur ou égal au diamètre de noyau du filetage extérieur (8) formé sur le segment fileté avant (5); et
H) le segment central (6) étant formé sans filetage.

2. Dispositif permettant la réduction, la compression et la fixation de fragments osseux, lequel comprend au moins une vis d'ostéosynthèse (1) selon la revendication 1,
**caractérisé en ce que**
A) le dispositif comprend un instrument d'implantation chirurgical (15) cylindrique ou prismatique qui présente un trou central traversant (17) s'étendant de manière coaxiale, une extrémité avant (18) et une extrémité arrière (19); et
B) le trou central (17) présente une partie élargie (24) s'étendant depuis l'extrémité avant (18) de l'instrument (15) jusqu'à une profondeur T et pourvue d'un filetage intérieur (20) complémentaire au filetage extérieur (9) formé sur le segment arrière (7) de la vis d'ostéosynthèse (1) et **en ce que** la profondeur T est supérieure ou égale à L (T ≥ L), L étant la longueur du segment fileté arrière (7) de la vis d'ostéosynthèse (1).
